# EUROPEAN PATENT APPLICATION

(11) **EP 1 878 739 A1**
(43) Date of publication of application: **16.01.2008**
(21) Application number: 06117221.9
(22) Date of filing: 14.07.2006
(51) Int. Cl.: C07K 1/22

(54) **One step IMAC (MCAC) purification of proteins**

(71) Applicant: LEK Pharmaceuticals d.d., 1526 Ljubljana (SI)
(72) Inventor: Menart, Viktor, 1370 Logatec (SI); Gaberç Porékar, Vladka, 1000 Ljubljana (SI); Preradov Vasle, Tatjana, 1000 Ljubljana (SI); Jesenko, Ana, 1360 Vrhnika (SI); Podobnik, Barbara, 1000 Ljubljana (SI)
(74) Representative: Kunic, Barbara

(57) **Abstract**

This invention relates to a process for isolation and/or purification of a protein of interest and to the use of IMAC as a capture and the main separation step.

## Description

### Field of the invention

The invention relates to use of Immobilized Metal-lon Affinity Chromatography (IMAC) for isolation and/or purification of a protein of interest.
In particular, this invention relates to a process for isolation and/or purification of Granulocyte-Colony-Stimulating Factor (G-CSF).

The principle of the present invention can be applied to a wide range of heterologous and homologous proteins as well as fusion proteins, expressed in a variety of host cells.

### Background of the invention

Whereas various techniques can be employed to produce high yields of a crude protein of interest, the isolation and purification of the protein is not a simple matter.
A typical isolation procedure comprise multi-stage processes comprising several purification steps *e.g.* different chromatographies, chromatofocusing, filtrations etc. In addition, in the known processes for isolation and/or purification of proteins the number of additional substances are used that could potentially contaminate the final product and furthermore, a number of toxic substances representing a pollution burden for environment e.g. denaturants, detergents, organic solvents (waste water) are normally used.
In the known purification processes where IMAC is used as capture and the key separation step, the improvement is needed, especially for use in the industrial scale. Furthermore, several described processes for isolation and/or purification of proteins involve very expensive chromatographic media, are inappropriate for cleaning-in-place and sanitization procedures or might represent a bottleneck in large-scale purifications (e.g. size exclusion chromatography, affinity matrices with immobilized monoclonal antibodies etc.).

The nature of the prior art procedure may lead to lower purity of a protein of interest, to higher costs of the protein production, to the environmental pollution and can be time-consuming. Therefore, in the view of the processes described above, there is a strong need for a simplified process for protein purification in a high yielding, cost-effective and, environmental-friendly matter.

Such simplified process for isolation and/or purification would comprise minimal number of purification steps, which would in addition have optimized technological parameters, capacity of chromatographic media, number of cleanings-in-place, sanitations cycles and similar. Thus, in the simplified process for purification the potential contaminants would be kept as low as possible during the process. In addition, in the simplified process for protein purification toxic substances should be kept as low as possible.
In the purification processes where Immobilized IMAC is used as capture and the main separation steps, the improvement is needed.
Simplified isolation and/or purification procedure shall be devoid of chromatographic steps that involve very expensive chromatographic media, are inappropriate for cleaning-in-place and sanitization procedures or might represent a bottleneck in large-scale purifications (e.g. size exclusion chromatography, affinity matrices with immobilized monoclonal antibodies etc.).

The present invention provides such a simplified process for isolation and/or purification of a protein of interest, in particular G-CSF.
The object of the present invention is to use IMAC, for the isolation and/or purification of a protein of interest, as both, a capture and the main separation step by combining two or more interaction and/or separation principles acting simultaneously. The approach of present invention can be applied to a wide variety of proteins.
In particular, the process for the isolation and/or purification of the present invention relates to isolation and/or purification of G-CSF. In this particular embodiment, IMAC as described above is used as the main chromatographic step. The process can additionally comprise a chromatographic step that serves as a polishing step and can be accomplished by various chromatographies. For final purity ultrafiltration / diafiltration can be applied.

The use of IMAC according to the present invention enables fast, environmental-friendly, good yield and cost-effective preparation of pharmaceutical-grade purity proteins.
In the case of G-CSF, the process of the present invention leads to over 98% RP-HPLC) purity of G-CSF and over 99% SE-HPLC purity of G-CSF with dimers and higher aggregates at the detection limit or below it.

### Summary of the invention

It is an object of the present invention to use IMAC as both, a capture and the main separation step by combining two or more interaction/separation principles acting simultaneously. Such use enables simpler purification and/or isolation of proteins, and provides biologically active proteins in highly purified and active form, as well as a pharmaceutical composition comprising the same.

According to the present invention it was surprisingly found that a simplified process for isolation and/or purification of a protein of interest can be achieved by a combination of various separation principles, comprising IMAC as the main chromatographic step functioning as both, a capture and the main separation step. Said main separation step involves two phases: the first phase uses a classical separation principle based on the specific protein affinity to the IMAC matrix with immobilized metal ions including binding at high pH and elution with lower pH buffer. In the second phase, a separation of the bound proteins is achieved using a combination of pH/salt gradient. The combination of two different interaction principles acting simultaneously on the same matrix results in better resolution and higher purity of the target protein.

In particular, the use of IMAC according to the present invention can be applied in the two-stage chromatographic process for isolation and/or purification of a protein of interest, e.g. G-CSF. The IMAC as described above can be used as the main chromatographic step leading to such a purity of a protein of interest, e.g. G-CSF that only one additional chromatographic step that serves as a polishing step can be used. This step can be accomplished by different chromatographies, e.g. affinity chromatography, hydrophobic interaction chromatography, reversed phase chromatography, anion exchange chromatography, cation exchange chromatography hydroxyapatite chromatography, and other chromatographies, depending on the nature of the target protein.
Ultrafiltration/diafiltration as the final isolation phase can be used for final buffer exchange and with a new function of final removing of protein dimers and other aggregates.

The use of IMAC according to the present invention enables preparation of pharmaceutical-grade purity proteins. In the case of G-CSF, the process for isolation and/or purification leads to over 98% RP-HPLC purity and over 99 % SE-HPLC purity of G-CSF with dimers and higher aggregates close to the detection limit or below it.

### Detailed description of the invention and preferred embodiments thereof

In the context of the present invention it has been surprisingly found that a unique way of using IMAC can lead to simpler, cost effective process for isolation and/or purification of proteins leading to high purity of proteins. Although the concept of the present invention can be applied to a wide variety of homologous and heterologous proteins, the present invention is particularly developed for the production of G-CSF.

In the present invention IMAC is used and functions as both, a capture and the main separation step by combining two or more separation principles acting simultaneously. A process for isolation and/or purification of proteins by using IMAC was described in W003051922, where IMAC was performed by using only one interaction principle with no additional separation principle acting simultaneously. In this case IMAC was performed by a step gradient elution and the whole chromatographic peak at 100% second elution buffer was pooled and used for further purification. In addition, IMAC was usually performed in such a way that binding and elution buffers contained high amounts of salt (usually NaCI or KCI) to reduce ionic interactions.
By using IMAC according to the present invention the stronger non-specific interactions than expected are surprisingly noticed. Specifically, G-CSF does not elute from the IMAC matrix by lowering pH if no salt is present in the elution buffer. As pure metal ion-protein affinity interactions are reduced at low pH and negligible at sufficiently low pH, this is probably due to residual non-specific interactions. In the prior art classical approaches these residual interactions are completely excluded by, e.g. using salt buffer.
In the present invention it is unexpectedly found out that contrary to the conventional approach, residual interactions can be efficiently used for improving separation power of IMAC. One way to achieve this additional separation is to apply combined elution gradients. This might be even more important in resolving structurally very similar product-related substances, because they in general have very similar affinity regarding the IMAC principle but are sufficiently different to be separated due to residual interactions with the chromatographic matrix.
The improvement is especially important on the industrial scale because it contributes to faster, environmental-friendlier and cost-effective production of a protein of interest.

In the main aspect of the invention, the use of IMAC for the isolation and/or purification of a protein of interest comprises:
1. a classical separation principle based on the specific protein affinity to the IMAC matrix with immobilized metal ions including binding at high pH and elution with lower pH buffer or by the addition of competing substances, such as imidazol, histidine or ammonium salts, and acting simultaneously
2. a further separation of the bound proteins by using a combination of pH/salt gradient (decreasing pH with simultaneous rising salt concentration) or a combination of competing substance/salt gradient (rising concentration of the competing substance with simultaneous rising salt concentration).

Therefore, in the main aspect the invention relates to a use of IMAC for the isolation and/or purification of a protein, wherein said use comprises:
- a classical separation principle based on the specific protein affinity to the IMAC matrix with immobilized metal ions, and acting simultaneously
- a further separation of the bound proteins by using a combination of pH/salt gradient or a combination of competing substance/salt gradient

The combination of two different interaction principles acting simultaneously on the same matrix results in better resolution and higher purity of the target protein.

The competing substance is preferably selected from the group consisting of imidazol, histidine or ammonium salts.

The purity indicated herein refers to RP-HPLC and SE-HPLC purity.

The term 'RP-HPLC purity' as used herein refers to the protein purity which is determined by RP-HPLC.

The term 'SE-HPLC purity' as used herein refers to the protein purity which is determined by SE-HPLC.

Accordingly, in the main aspect of the present invention, there is provided a use of IMAC for the isolation and/or purification of a protein of interest, comprising:
a) loading a solution or mixture, which comprises a protein, on the IMAC support
b) selective binding of biologically active form of a protein to the IMAC support at high pH, optionally washing the IMAC column,
c) eluting protein and non-protein contaminants from the IMAC column by lower pH buffer or by the addition of the competing substances such as histidine, imidazole or ammonium salts
d) eluting the biologically active form of a protein of interest from the IMAC column by using combined gradient (pH/salt or competing substance/salt gradient)
e) identification of fractions to be pooled or rejected (using SDA-PAGE, RP-HPLC and SE-HPLC)
f) pooling of the fractions

Therefore, in the main aspect the invention relates to a use of IMAC for the isolation and/or purification of a protein comprising:
- loading a solution or mixture, which comprises a protein, on the IMAC support
- selective binding of biologically active form of a protein to the IMAC support at high pH, optionally washing the IMAC column,
- eluting protein and non-protein contaminants from the IMAC column by lower pH buffer or by the addition of competing substances
- eluting the biologically active form of a protein of interest from the IMAC column by using combined gradient
- identification of fractions to be pooled or rejected
- pooling of the fractions

The competing substance is preferably selected from the group consisting of imidazol, histidine or ammonium salts.

The process of the present invention can be used in case of purification and/or isolation of proteins, which are selected from the group consisting of granulocyte - colony stimulating factor (G-CSF) and G-CSF derivatives such as methionyl G-CSF (Met-G-CSF), glycosylated, enzymatically or chemically modified (e. g. pegylated) G-CSF, G-CSF analogues and the fusion proteins which comprise G-CSF, other fusion proteins (e.g. with albumin, with Fc), His Tagged proteins, interferons (IFN), such as INF-beta 1b, IFN-beta 2b, IFN-gamma 1b, interleukins (IL), such as IL-2 and IL-4, granulocyte macrophage-colony stimulating factor (GM-CSF), macrophage-colony stimulating factor, (M-CSF), epidermal growth factor (EGF), human serum albumin (HSA), deoxyribonuclease (DNAse), fibroblast growth factor (FGF), tumour necrosis factor alpha (TNF alpha) and tumour necrosis factor beta (TNF-beta) and further comprises other proteins.
Preferably, the selected protein is G-CSF.

Accordingly, in the particular aspect of the present invention, there is provided a use of IMAC for the isolation and/or purification of G-CSF, comprising :
(1) loading a solution or mixture, which comprises G-CSF, on the IMAC support
(2) selective binding of biologically active form of G-CSF to the IMAC support at high pH, optionally washing the IMAC column,
(3) eluting protein and non-protein contaminants from the IMAC column by lower pH buffer or by the addition of the competing substances such as histidine, imidazole or ammonium salts,
(4) eluting the biologically active form of G-CSF of interest from the IMAC column by using combined pH and salt gradient (pH/salt or competing substance/salt gradient)
(5) identification of fractions to be pooled or rejected (using SDA-PAGE, RP-HPLC and SE-HPLC)
(6) pooling of the fractions

Therefore, in the main aspect the invention relates to a use of IMAC for the isolation and/or purification of G-CSF comprising:
- loading a solution or mixture, which comprises G-CSF, on the IMAC support
- selective binding of biologically active form of G-CSF to the IMAC support at high pH, optionally washing the IMAC column,
- eluting protein and non-protein contaminants from the IMAC column by lower pH buffer or by the addition of competing substances
- eluting the biologically active form of G-CSF from the IMAC column by using combined gradient
- identification of fractions to be pooled or rejected
- pooling of the fractions

In addition to the highly effective process of the present invention, the process can also be all along performed under native conditions. Thus renaturation steps can be omitted.

The term 'native conditions' used herein refers to the conditions by which the molecule (a protein, e.g. G-CSF) preserves the native conformation and the biological activity.

In one embodiment the use of IMAC according to the present invention is applied in the process for isolation and/or purification of G-CSF. In this case IMAC is applied as the first chromatographic step. After said first chromatographic step 95.5% RP-HPLC purity of G-CSF and over 99 % SE-HPLC purity of G-CSF with dimers and higher aggregates close to the detection limit or below it is achieved.

Accordingly, in a further purification process of a protein of interest only polishing and removal of dimers and other aggregates can be needed.

The process for isolation and/or purification of proteins of the present invention can additionally comprise further polishing. Depending on the nature of the protein of interest, further polishing of the protein according to the present invention can be accomplished by using a second chromatographic step. Said chromatographic step can be selected from the group consisting of an affinity chromatography, hydrophobic interaction chromatography, reversed phase, anion exchange chromatography, cation exchange chromatography or hydroxyapatite chromatography.
In the case of isolation and/or purification of G-CSF, cation exchange chromatography can be preferentially applied as the second chromatographic step.

For final exchange of buffer and removal of protein dimers and other aggregates ultrafiltration or diafiltration can be used as the final isolation and/or purification step in the process of the present invention. Ultrafiltration or diafiltration can replace the gel filtration step which can be a bottle neck in the industrial preparation of proteins.

Accordingly, in one aspect the present invention relates to a process for the two-chromatographic stage isolation and/or purification of a protein of interest, which process consists of the following chromatographic steps:
(a) IMAC used as described above, and
(b) a chromatography

Accordingly, in one aspect the present invention relates to a process for the two-chromatographic stage isolation and/or purification of a protein of interest, which process consists of the following chromatographic steps:
(a) IMAC used as described above, and
(b) a chromatography, selected from the group consisting of an affinity chromatography, hydrophobic interaction chromatography, reversed phase, anion exchange chromatography, cation exchange chromatography or hydroxyapatite chromatography

Therefore, in one aspect the present invention relates to the use of IMAC as described above in the process for isolation and/or purification of a protein, wherein IMAC is used as the main chromatographic step.

Therefore, in one aspect the present invention relates to the use of IMAC as described above in the process for isolation and/or purification of a protein, wherein IMAC is used as the first chromatographic step.

Optinally, the two-chromatographic process of the present invention can further comprise:
(c) ultrafiltration of diafiltration

In another aspect, the process for isolation and/or purification of G-CSF of the present invention consists of:
(a) IMAC used as described above, and
(b) cation exchange chromatograpy, and
(c) ultrafiltration or diafiltration

No additional steps (e.g. concentration, changing of buffers, filtrations, freezing, etc) are needed.

The process for purification and/or isolation of proteins e.g. G-CSF of the present invention can be transferred to industrial scale and to the production of biologically active proteins. In the case of G-CSF 98% RP-HPLC purity of G-CSF and over 99 % SE-HPLC purity of G-CSF with dimers and higher aggregates close to the detection limit or below it is achieved.

The process for purification and/or isolation of protein of the present invention results in the production of biologically active proteins which can be suitable for clinical use in medicine.

Exemplary, the process leads to the production of biologically active G-CSF suitable for clinical use in medicine.

The biologically active protein e.g. G-CSF obtained by the process for the purification and/or isolation of the present invention is suitable for the preparation of pharmaceutical composition, which comprises the therapeutically effective amount of biologically active protein e.g. G-CSF and is suitable for clinical use.

The term 'therapeutically effective amount' used herein refers to the amount of a biologically active protein e.g. G-CSF which has the therapeutic effect of biologically active protein e.g. G-CSF.

The possibility of maintaining the active form of a protein, e.g. G-CSF in a short purification and isolation process contributes not only to an improved yield, but also to an improved purity and effectiveness of the biologically active protein e.g. G-CSF and the pharmaceutical composition containing it.

Suitable pharmaceutically acceptable auxiliary substances include suitable diluents, adjuvants and/or carriers useful in protein e.g. G-CSF therapy.

Exemplarily, biologically active G-CSF which was obtained by using the process of the present invention, particularly when performing the additional steps of cationic exchange chromatography can be used for preparation of medicaments, which are indicative for G-CSF.

The pharmaceutical composition containing the pure and biologically active G-CSF obtained by the process of the invention can thus be administered, in a manner known to those skilled in the art, to patients in a therapeutically amount which is effective to treat the above mentioned diseases.

The process for isolation and/or purification of proteins of the present invention is most preferably maintained under native conditions and is described in details in the following.

The process can begin with the sample preparation. The samples can be prepared by any one of the following processes: direct extraction of the whole cells expressing the target protein, homogenisation of the cells expressing the target protein either in the soluble form in the cytoplasm or in the form of inclusion bodies, denaturing solubilization of "classical" inclusion bodies (using chaotropes such as 6-8 M urea or 4-6 M guanidinium hydrochloride, high pH buffers, surfactants at high concentration, etc) and subsequent renaturation (performed by dilution, denaturant removal e. g. by dialysis, buffer exchange etc.). The samples can also be prepared by mild solubilization of "non-classical" inclusion bodies (containing a high percentage of correctly folded target protein or its precursor) under native conditions e.g. urea in non-denaturing concentrations (1-2 M, preferably in a buffer at a pH of below 10 and more preferably at a pH of about 8.0), N-lauroyl sarcosine in non-denaturing concentrations (0.05-0.25% (m/v)), low concentrations of Zwittergents, different non-detergent sulfobetaines (NDSB), betaine, sarcosine, carbamoyl sarcosine, taurine, dimethylsulfoxide (DMSO) and higher concentrations of buffers (preferably at a pH being maintained at below 10 and more preferably about 8.0), for example: HEPES, HEPPS, MES, ACES. Preferably, N-lauroyl sarcosine, NDSB and DMSO are used. Most preferably N-lauroyl sarcosine in the concentration range from 0.1% to 0.25% (m/v) is used.
In the case of cytoplasmatic expression, the soluble protein solution after solid/liquid phase separation of the cell homogenate can be directly used for chromatographic isolation/purification.

In one embodiment, the samples can be prepared in a buffer that can be directly used for the first chromatographic step IMAC.

In the case of G-CSF the mild extraction of 'non-classical inclusion bodies' with NDSB is preferably used. NDSB is non-denaturing for the protein and does not interfere with chromatographic matrix. Most preferably, NDSB in the concentration range from 0.1 % to 0.25% (m/v) is used.

The IMAC support comprises a solid phase material and a metal ion chelate bound to the solid phase material. Conventional solid phase materials can be suitably used, such as Sepharose, Fractogel and other gel support materials. The metal ion chelate bound to the IMAC support is suitably selected from metal ions preferably having two valencies, especially transition metal ions. Hg is less suitable in view of its toxicity and its tendency to be leached out of the IMAC column. Preferably, the metal ion chelates, being bound to the IMAC support, comprise: M(II)-iminodiacetate (IDA), M(II)-nitrilotriacetic acid (NTA), M(II)-carboxymethylaspartate etc., where M presents Zn, Cu, Co, Ni etc. Most preferably Zn(II) -IDA, Ni(II)-IDA and Ni(II)-NTA are used.
The eluate resulting from the first elution from the IMAC column can also be used as loading solution or mixture for IMAC. The pH of the eluate should be adjusted, e.g. by addition of a NaOH solution or a high pH buffer solution, to the pH range from 6.5 to 9.0. The eluate can be then reloaded to the same IMAC support such as, e.g., Zn(II)-IDA, Ni(II)-IDA, Ni(II)-NTA or to a different IMAC support. The combination with other immobilised metal ions is also possible (e.g. Zn(II), Cu(II), Co(II), Ni(II) etc..) and enables better separation and removal of specific host proteins.

Regardless to the preparation and the origin of the loading solution, the pH of the loading solution is in the range from 6.5 to 9.0. Preferred pH of loading solution is from 7.0 to 8.5, the mostly preferred pH is from 7.8 to 8.2.
Various buffers, which can maintain pH in the range from 6.5 to 9.0 can be used for loading and binding of G-CSF to the IMAC support and are selected from the group consisting of: phosphate, acetate, hydroxymethylaminomethan (Tris)/HCI, Tris/acetate, citrate and other buffers providing a pH of from 6.5 to 9.0. Preferably, Tris/HCl is used.
Tris/HCl buffer is preferably used in the concentration range from 5 to 50 mM, most preferably in the range from 10 to 40 mM.
After binding to the support the process is continued by washing of the column and elution of proteins from the column. Elution can be performed by using either a discontinuous step gradient or linear gradient by descending pH or competitive elution at high pH (e.g. with imidazole, histidine, ammonium chloride and similar).
The term 'linear gradient' used herein refers to washing of chromatographic column by a solution which composition is changed in a way that the proportion of one buffer (or one component of the buffer) is increased linearly, whereas the proportion of the other buffer (or another component of the buffer) is decreased linearly.
The term 'discontinuous step gradient' used herein refers to washing of chromatographic column by a solution, which is composed of certain proportion of one buffer (or one component of the buffer) and certain proportion of another buffer (or another component of the buffer) for a determined time period. The proportions of both buffers are rapidly (suddenly) changed and the column is washed by another determined time period. The composition of the solution (change of buffer proportions or component proportions) is not changed linearly.

The term 'competitive elution' used herein refers to elution at the pH of the binding buffer where the competitor molecules, such as imidazole, histidine, ammonium chloride etc., in the elution buffer bind to the metal chelated matrix by themselves and thus displace the protein molecules.

Preferably, discontinuous step gradient, resulting to elution at low pH is used. Namely, high pH could cause the activation of cysteine residues and the formation of dimers. Stability of G-CSF is also high at low pH.

Several elution buffers can be used for discontinuous step or linear washing and elution and are selected from the group consisting of: acetate, Tris/acetate, phosphate, citrate and other suitable buffers. The pH range for the elution can be from 3.0 to 5.0, preferably 3.5 to 4.5. By the discontinuous step gradient, the pH is rapidly changed from the order of the loading pH to the order of elution pH, such as from 7.0 to 4.0, and the isoelectric point is thus jumped over, avoiding the precipitation of the protein. Namely, environment with the pH of the protein isoelectric point can cause its precipitation.

The eluate obtained from the IMAC column can be loaded to different chromatographic columns, depending on the nature of protein, and are selected from the group consisting of affinity chromatography, hydrophobic chromatography, reversed phase chromatography, anion exchange chromatography, cation exchange chromatography and hydroxyapatite chromatography.
In one embodiment the eluate obtained from the IMAC column can be loaded directly to the cationic exchange chromatography column, without any additional intermediate steps being required. Various cation exchange chromatography supports can be used and may be selected from the group consisting of: SP Sepharose FF, SP Sepharose HP, CM Sepharose FF, TSK gel SP-5PW, TSK gel SP-5PW-HR, Toyopearl SP-650M, Toyopearl SP-650S, Toyopearl SP-650C, Toyopearl CM-650M, Toyopearl CM-650S, Macro-Prep High S support, Macro-Prep S support, Macro-Prep CM support etc. Preferably, Toyopearl SP-650S or TSK gel SP-5PW are used.
pH range of the loading solution for cationic exchange chromatography is in the range from 3.0 to 5.8, preferably in the range from 4.0 to 5.0.
The salt concentration in the loading solution for cationic exchange chromatography has to be low enough to enable the binding, which also depends on pH of the solution.

Various buffers with the pH range from 3.0 to 5.8 can be used for loading and binding to the support for cationic exchange chromatography and may be selected from the group consisting of: acetate, citrate, Tris/HCl, Tris/acetate, phosphate, succinate, malonate, 2-(N-morfolinoethansulfonate) (MES) and other buffers. Preferably, acetate buffer is used.
Acetate buffer can be used in the concentration range from 10 to 60 mM, preferably in the concentration range from 10 to 30 mM.
In the cationic exchange chromatography, the column loading is followed by washing of the column and the elution of the proteins from the column. The elution occurs due to increased ionic strength after the addition of high concentration of salt in buffer solution. Discontinuous step gradient, linear gradient and a suitable combination of step and linear gradient can be used.
Elution buffers, which can be used for washing and elution, may be selected from the group consisting of: acetate, citrate, Tris/HCl, Tris/acetate, phosphate, succinate, malonate, MES and other suitable buffers with addition of salts such as NaCl or KCI. Ionic strength and salt concentration, by which the elution is achieved, depends on the pH of the buffer solution. The higher is pH of the buffer, the lower ionic strength is needed for the elution of the proteins from the column.

If desired, the eluate obtained from the IMAC or, preferably, after the consecutive cationic exchange chromatography column can be loaded directly to the ultrafiltration / diafiltration, without any additional intermediary steps being required.

In the eluate, monomeric, biologically active, correctly folded G-CSF is obtained with a RP-HPLC purity of G-CSF of greater than 98% and SE-HPLC purity of G-CSF of greater than 99% and biological activity of about 1x10⁸ IU/mg.

The following examples are given for the purpose of illustrating various embodiments of the invention and are not meant to limit the present invention in any fashion.

### Examples

### Example 1: IMAC (Chelating Sepharose FF, Pharmacia)

Inclusion bodies from G-CSF expressing *E. coli* are solubilized under native conditions (either by 0.2% NDSB (NDSB 195, NDSB 201, NDSB 211, NDSB 256) or 0.2% N-lauroyl sarcosine (NLS) in P1. In the case of NLS solubilization, the detergent must be removed (for example using an ion exchange resin) prior to IMAC step while NDSBs are compatible with IMAC and do not interfere with separation, therefore, no removal step is needed.
The obtained protein solution (approximately 150 mg) with an approximate concentration of 1.8 mg/ml is applied to the Amersham chromatographic XK 26/20 column loaded with Chelating Sepharose fast flow (h=10 cm, d=2.6 cm, V=50 ml) which was pre-charged with Zn(II) ions. After loading, the column is washed with P2 buffer for 20 minutes (2 Vc) followed by a 15 minute-wash (1.5 Vc) with a mixture of buffers P2 and P3 at the volume ratio of 65:35. Afterwards, P4 buffer is pumped through the column for 20 minutes (2Vc), followed by a shallow linear gradient of P5 buffer (from 100% P4 to 40% P4 and 60% P5 in 47 minutes (4.7 Vc)). Final elution is accomplished by 100% P5 in 25 min (2.5 Vc). The whole chromatographic procedure is performed at a constant flow rate of 5 ml/min (56 cm/h). The gradient used is

The typical chromatogram is composed of three major peaks:
Peak A in the flow-through represents mostly aggregated G-CSF and host cell proteins, which do not bind to IMAC matrix.
Peak B eluted during the linear gradient belongs to the monomeric biologically active G-CSF. Careful pooling of the fractions on the basis of RP-HPLC, SE-HPLC and
SDS-PAGE (under reducing and non-reducing conditions) analyses results to improved purity of the IMAC-pool (Table 1), which is directly used in the next, polishing chromatographic step. For further purification, the central part of peak B is pooled, based on the results of RP-HPLC, SE-HPLC and SDS-PAGE analyses.
Peak C eluted at 100% P5 is more than 90% pure disulfide-linked dimer of G-CSF. based on the Gradient No. 4 from the Table 1.

**Table 1: Step by step improvement of combined pH & salt gradient and achieved purity of the IMAC pool.**

| Gradient No. | Buffer A | Buffer B | Gradient description | Purity of IMAC-pool by RP-HPLC | Purity of IMAC-pool by SE-HPLC |
|---|---|---|---|---|---|
| 1 | 20 mM Tris/HCl, pH 7.0 | 20 mM acetic acid, 150 mM NaCl, pH 4.0 | Linear in 7 CV | 88 % | 1.9 % dimer |
| | | | | | 0.2 % oligomer |
| 3 | 20 mM Tris/HCl, pH 7.0 | 20 mM acetic acid, 100 mM NaCl, pH 4.0 | Linear in 7 CV | 93 % | 0.34 % dimmer |
| | | | | | 0% oligomers |
| 4 | 20 mM Tris/HCl, pH 7.0 | 20 mM acetic acid, 100 mM NaCl, pH 4.0 | Linear in 8 CV | 95.5% | 0.36 % dimmer |
| | | | | | 0% oligomers |

### Composition of buffers:

P1: 40mM Tris/HCL, pH 8.0
P2: 20 mM Tris/HCl, 150 mM NaCl, pH 8.0
P3: 20 mM CH₃COOH/NaOH, 150 mM NaCl, pH 4.0
P4: 20 mM Tris/HCl, pH 7.0
P5: 20 mM CH₃COOH/NaOH, 100 mM NaCl, pH 4.0

### Example 2: Process for the purification and/or isolation of G-CSF, including additional purification steps

IMAC pool (the central part of peak B) is diluted with the same volume of P6 buffer (20 mM CH₃COOH, pH 4,0) and directly applied to the chromatographic column HR10/10 (Amersham), loaded with chromatographic support Toyopearl 650S (35 µm; TosoHaas) to the height of 10 cm (h=10 cm, d=1,0 cm, V= 8 ml). Loading of the sample and elution from the column are performed at a constant flow of 2 ml/min. After loading the sample, the column is washed with P7 buffer for 6 min (1.5 Vc). Elution is accomplished by a linear gradient of P8 buffer from 0% to 18% P8buffer in 26 min (6.5 Vc) (from 100% to 82% P7 buffer). Afterwards, the column is washed with the mixture of P7 and P8 buffers in a volume ratio of 82:18 for 12 min (3 Vc) and then for 8 min (2Vc) with 100% P8 buffer. Fractions of the main chromatographic peak are analysed by SDS-PAGE, RP-HPLC and SE-HPLC and pooled on the basis of these analyses. The buffer is exchanged by ultrafiltation/diafiltration. The final, sterile-filtered G-CSF has over 99% SE-HPLC purity, over 98% RP-HPLC purity, and biological activity of approximately 1 x 10⁸ IU/mg, which corresponds to the biological activity of the standard.
Composition of buffers:
P6: 20 mM CH₃COOH, pH 4,0
P7: 20 mM CH₃COOH, pH 5,5
P8: 20 mM CH₃COOH, 500 mM NaCl, pH 5,5

### Example 3: In vitro G-CSF biological activity assay

Biological activity of G-CSF is determined by the method based on stimulation of cellular proliferation (NFS-60 cells) using the known method (Hammerling, U. et al. in J Pharm Biomed Anal 13, 9-20 (1995)) and the use of international standard Human recombinant G-CSF (88/502, yeast cell derived; NIBSC Potters Bar, Hertfordshire, UK; see Mire-Sluis,A.R. et al. v J lmmunol Methods 179, 117-126 (1995)

## Claims

**1.** A use of IMAC for the isolation and/or purification of a protein, wherein said use comprises:
- a classical separation principle based on the specific protein affinity to the IMAC matrix with immobilized metal ions, and acting simultaneously
- a further separation of the bound proteins by using a combination of pH/salt gradient or a combination of competing substance/salt gradient

**2.** A use of IMAC for the isolation and/or purification of a protein comprising:
- loading a solution or mixture, which comprises a protein, on the IMAC support
- selective binding of biologically active form of a protein to the IMAC support at high pH, optionally washing the IMAC column,
- eluting protein and non-protein contaminants from the IMAC column by lower pH buffer or by the addition of competing substances
- eluting the biologically active form of a protein of interest from the IMAC column by using combined gradient
- identification of fractions to be pooled or rejected
- pooling of the fractions

**3.** The use of IMAC according to claim 2 wherein the protein is G-CSF.

**4.** The use of IMAC according to claim 2 wherein the combined gradient is selected from the group consisting of pH/salt and competing substance/salt gradient.

**5.** The use of IMAC according to claims from 1 to 4 in the process for isolation and/or purification of a protein, wherein IMAC is used as the main chromatographic step.

**6.** The use of IMAC according to claims from 1 to 4 in the process for isolation and/or purification of a protein, wherein IMAC is used as the first chromatographic step.

**7.** The process for isolation and/or purification of a protein according to claim 6, wherein the process further comprises a chromatography.

**8.** The process according to claim 7 wherein the chromatography is selected from the group consisting of an affinity chromatography, hydrophobic interaction chromatography, reversed phase, anion exchange chromatography, cation exchange chromatography or hydroxyapatite chromatography

**7.** The process according to claim 8 wherein the chromatography is cation exchange chromatography.

**8.** The process according to claim 8 wherein the process further comprises ultrafiltration or diafiltration.

**9.** The process according to claims from 5 to 8 wherein the protein is G-CSF

**10.** A pharmaceutical composition obtained by the process according to any one of claims from 6 to 9.
